# EUROPÄISCHE PATENTSCHRIFT

(11) **EP 3 182 938 B1**
(45) Veröffentlichungstag und Bekanntmachung des Hinweises auf die Patenterteilung: **15.08.2018**
(21) Anmeldenummer: 15753685.5
(22) Anmeldetag: 21.08.2015
(51) Int. Cl.: A61F 5/01, A61F 5/02, A61F 5/32, A61H 1/00, A61H 7/00, A61H 39/04, A61H 11/00, A61F 13/14

(54) **RÜCKEN- ODER BECKENBANDAGE**
BACK OR PELVIC BELT
BANDAGE POUR LE DOS OU LE BASSIN

(30) Priorität: 22.08.2014 DE 102014012654
(43) Veröffentlichungstag der Anmeldung: 28.06.2017
(73) Patentinhaber: Bauerfeind AG, 07937 Zeulenroda-Triebes (DE)
(72) Erfinder: HESS, Heinrich, 66271 Kleinblittersdorf (DE)
(74) Vertreter: Wohlfahrt, Jan Günther
(86) Internationale Anmeldenummer: PCT/EP2015/069272
(87) Internationale Veröffentlichungsnummer: WO 2016/026968

(56) Entgegenhaltungen:
- WO-A1-2012/131298
- DE-A1-102011 000 953
- DE-A1-102012 017 722
- DE-T2- 60 319 402
- US-A- 3 783 879
- US-A- 4 794 916
- US-A- 5 447 498

## Beschreibung

Die vorliegende Erfindung betrifft eine Rücken- oder Beckenbandage mit benachbarten Pelotten, die auf der Innenseite eines Tragstücks im Rückenbereich der Bandage angebracht sind, wobei an den gegenüberliegenden Randbereichen des Tragstücks jeweils ein Schließbandstück zum Anlegen der Bandage durch Zusammenfassen der Enden der beiden Schließbandstücke ansetzt, wobei dem Tragstück zwei Zuggurte zugeordnet sind, die derart geführt sind, dass die Pelotten bei Spannung der Zuggurte sich annähern und auf das betreffende Körperteil einen von einer auf die Zuggurte wirkenden Zugkraft abhängigen Druck ausüben.

Eine derartige Bandage ist in der DE 10 2011 000 953 A1 offenbart. Dort sind zwei Zuggurte vorgesehen, die auf der Außenseite des Tragstücks befestigt sind. Die Gurtführung zur Bauchseite der Bandage ist dabei entweder über Kreuz vorgesehen, oder aber über ein Umlenkstück. Die erste Ausführungsform hat den Nachteil, dass eine Person, die die Bandage anlegt, beim Anziehen der Zuggurte mit dem rechten Zuggurt auf die linke Pelotte einwirkt und mit dem linken Zuggurt auf die rechte Pelotte einwirkt, so dass die Bandage leicht falsch angezogen werden kann, was insbesondere bei einseitigen, beispielsweise nur rechts- oder nur linksseitigen Schmerzzuständen nachteilig ist. Bei der zweiten Ausführungsform reiben die umgelenkten Zuggurte beim Anziehen aneinander.

Der vorliegenden Erfindung liegt das technische Problem zugrunde, eine Rücken- oder Beckenbandage bereitzustellen, die die Nachteile aus dem Stand der Technik überwindet.

Weiterhin soll eine Rücken- oder Beckenbandage bereitgestellt werden, bei der die Zuggurte eine direktere Krafteinleitung auf die Pelotten ermöglichen.

Auch soll eine Rücken- oder Beckenbandage bereitgestellt werden, die einen flachen und kompakten Aufbau des Zuggurtsystems ermöglicht.

Die vorliegende Erfindung löst das ihr zugrundeliegende technische Problem durch den Gegenstand von Anspruch 1.

Die vorliegende Erfindung löst das ihr zugrunde liegende technische Problem insbesondere durch eine Rücken- oder Beckenbandage mit benachbarten Pelotten, die auf der Innenseite eines Tragstücks im Rückenbereich der Bandage angebracht sind, wobei an den gegenüberliegenden Randbereichen des Tragstücks jeweils ein Schließbandstück zum Anlegen der Bandage durch Zusammenfassen der Enden der beiden Schließbandstücke ansetzt, wobei dem Tragstück zwei Zuggurte zugeordnet sind, die derart geführt sind, dass die Pelotten bei Spannung der Zuggurte sich annähern und auf das betreffende Körperteil einen von einer auf die Zuggurte wirkenden Zugkraft abhängigen Druck ausüben, wobei die Zuggurte auf der Innenseite des Tragstücks im Bereich der Pelotten mit dem Tragstück verbunden sind.

Es zeigte sich, dass eine Verbindung der Zuggurte auf der Innenseite des Tragstücks im Bereich der dort angebrachten Pelotten in vorteilhafter Weise eine direkte Krafteinleitung von den Zuggurten auf den Bereich der Pelotten und die Pelotten selbst ermöglicht. Die Zuggurte können somit direkt bei den Pelotten enden und dort wirken. Durch diese technische Lösung können auch Schmerzzustände in nur einem Iliosakralgelenk günstig beeinflusst werden.

In einer bevorzugten Ausführungsform verläuft jeweils ein Zuggurt auf der Außenseite von jeweils mindestens einem Teilbereich eines Schließbandstücks.

Bevorzugt weist das Tragstück mindestens eine Öffnung auf, durch die die Zuggurte auf die Innenseite des Tragstücks geführt werden.

Bevorzugt weist das Tragstück zwei Öffnungen auf, wobei durch jeweils eine Öffnung ein Zuggurt auf die Innenseite des Tragstücks geführt wird.

In einer bevorzugten Ausführungsform verlaufen die Zuggurte teilweise auf der Außenseite des Tragstücks und das Tragstück weist mindestens eine Öffnung auf, durch die die Zuggurte auf die Innenseite des Tragstücks geführt werden.

Die mindestens eine Öffnung, insbesondere die zwei Öffnungen, ermöglicht es in vorteilhafte Weise, dass die Zuggurte von der Innenseite auf die Außenseite des Tragstücks geführt werden können und somit zum einen eine erfindungsgemäße Verbindung der Zuggurte auf der Innenseite des Tragstücks im Bereich der Pelotten ermöglicht wird und andererseits gleichzeitig eine herkömmliche Führung der Zuggurte und etwaiger Spannbänder auf der Außenseite, insbesondere im vorderen Bereich der Bandage, ermöglicht wird, so dass der Träger der Bandage die Zuggurte oder etwaige Spannbänder ohne Weiteres greifen und verwenden kann. Darüber hinaus hat die mindestens eine Öffnung, durch die die Zuggurte von der Außenseite auf die Innenseite des Tragstücks geführt werden, den Vorteil, dass hier eine Umlenkung erfolgen kann, die eine einfachere, leichter gleitende und symmetrische Krafteinleitung nach dem Seilzugprinzip ermöglicht. Auch laufen die Zuggurte trotz der Umlenkung in vorteilhafter Weise beim Festziehen kaum gegenläufig und reiben nicht aneinander, da der gegenläufige Abschnitt eines Zuggurtes auf der Innenseite der Bandage verlaufen kann.

Daher befindet sich die mindestens eine Öffnung in bevorzugter Weise bei den erfindungsgemäß bevorzugten Umlenkstücken.

Bevorzugt wird der erste Zuggurt durch eine erste Öffnung im Tragstück auf die Innenseite des Tragstücks geführt und der zweite Zuggurt wird durch eine zweite Öffnung im Tragstück auf die Innenseite des Tragstücks geführt.

Bevorzugt ist die mindestens eine Öffnung ein Schlitz im Tragstück. Bevorzugt sind die zwei Öffnungen jeweils ein Schlitz im Tragstück, besonders bevorzugt mit einer Umrandung. Diese dient in vorteilhafter Weise als Verstärkung, so dass die Schlitze nicht ausreißen können.

Bevorzugt sind die Zuggurte je über ein an dem Tragstück angebrachtes Umlenkstück unter Richtungsumkehr von dem Bereich der Pelotten zu dem jeweiligen Randbereich des Tragstücks geführt.

Bevorzugt sind die Umlenkstücke auf der Außenseite des Tragstücks befestigt.

Bevorzugt bildet eine in der Mitte des Tragstücks befestigte Umlenköse die zwei Umlenkstücke.

Bevorzugt ist vorgesehen, dass der erste Zuggurt durch einen ersten Schlitz von der Innenseite der Bandage auf die Außenseite geführt wird und der zweite Zuggurt durch einen zweiten Schlitz von der Innenseite der Bandage auf die Außenseite der Bandage geführt wird und die Zuggurte dann über eine in der Mitte des Tragstücks befestigten Umlenköse umgelenkt werden und auf der Außenseite der Bandage auf die Vorderseite der Bandage geführt werden.

Dies ermöglicht eine besonders flach gestaltete Ausführungsform im Rückenbereich der Bandage.

In einer bevorzugten Ausführungsform sind die Pelotten an dem Tragstück reversibel anbringbar, besonders bevorzugt durch eine Klettverbindung. Diese hat den Vorteil, dass die Pelotten individuell positioniert werden können und auch die durch die Zuggurte wirkende Krafteinleitung damit individualisiert werden kann. Bevorzugt sind die Pelotten in dem Bereich des Tragstücks anbringbar, an dem die Zuggurte mit dem Tragstück verbunden sind. Bevorzugt sind also die Klettverbindungselemente für die Pelotten am Tragstück in dem Bereich, an dem die Zuggurte mit dem Tragstück verbunden sind.

Dem Fachmann sind geeignete Pelotten bekannt. Bevorzugt weisen die Pelotten Noppen auf. Diese Noppen wirken bei Bewegung der Pelotten im Sinne einer Friktionsmassage auf die schmerzbeeinflussenden "Triggerpunkte" in den Weichteilen, zum Beispiel in der Muskulatur. In einer Ausführungsform kann vorgesehen sein, dass die Pelotten mit Heizelementen oder Kühlelementen versehen werden können. Durch eine bevorzugte reversible Anbringbarkeit der Pelotten können solche Heizelemente oder Kühlelemente leicht ausgetauscht oder ersetzt werden.

Um an der Rücken- beziehungsweise Beckenbandage unterschiedliche Pelotten anbringen zu können, wird das Tragstück in bevorzugter Ausführungsform mit einer Klettverschlussoberfläche versehen, die zu den entsprechenden Klettverschlussoberflächen der Pelotten passt, so dass verschiedene Pelotten über die Klettverschlüsse in einfacher Weise ausgetauscht werden können.

Die Zuggurte sind im Bereich der Pelotten mit dem Tragstück verbunden. Dabei kann vorteilhafterweise vorgesehen sein, dass die Zuggurte im Bereich der Pelotten mit dem Tragstück verbunden sind, insbesondere an der gleichen Position wie die Pelotten mit dem Tragstück verbunden sind, was eine besonders gute Krafteinleitung auf die Pelotten ermöglicht.

Bevorzugt ist jeweils ein Zuggurt an jeweils einem Befestigungsbereich einer Pelotte mit dem Tragstück verbunden, insbesondere dort direkt mit dem Tragstück verbunden.

In einer alternativen Ausführungsform sind die Zuggurte über jeweils über ein am Tragstück befindliches fahnenartiges Befestigungselement mit dem Tragstück verbunden. Ein solches fahnenartiges Befestigungselement, auch als Flappe bekannt, ermöglicht eine besonders gute Krafteinleitung der Zugkräfte eines Zuggurtes direkt auf den Bereich einer Pelotte.

Die Spannung der Zuggurte lässt sich vorteilhaft dadurch herbeiführen, dass jeder Zuggurt jeweils zu einem Ösenelement in Form einer Zugöse führt und in dieser endet, durch die jeweils ein Spannband geführt ist, das mit seinem einen Ende zu einem Schließspannstück und mit seinem anderen Ende frei zum Erfassen derart geführt ist, dass bei Spannung der beiden Spannbänder das Tragstück sich zusammenzieht und die Pelotten einander annähern. Die freien Enden der beiden Spannbänder lassen sich auf diese Weise von Hand ziehen, womit die Spannbänder über die Umlenkösen einen Zug ausüben, der sich auf die Zuggurte fortsetzt und damit eine gegenläufige Spannung auf das Tragstück ausübt, durch die das Tragstück zusammengezogen wird und die Pelotten einander annähern.

Bevorzugt weisen das Tragstück und die am Tragstück angrenzenden Bereiche der Schließbandstücke die gleiche Elastizität auf.

Bevorzugt weisen das Tragstück und mindestens die am Tragstück angrenzenden Hälften der Schließbandstücke die gleiche Elastizität auf. Bevorzugt weisen das Tragstück und die am Tragstück angrenzenden Hälften der Schließbandstücke die gleiche Elastizität auf.

Es zeigte sich überraschender Weise, dass bei der erfindungsgemäßen Ausführungsform der Zuggurte es nicht nötig ist, dass das Tragstück flexibler sein muss als die angrenzenden Schließbandstücke, wie es bislang beispielsweise in der auf der DE 10 2011 000 953 A1 beruhenden SacroLoc® Orthese der Bauerfeind AG, Zeulenroda-Triebes, Deutschland verwirklicht ist. Bei solchen Rücken- oder Beckenbandagen aus dem Stand der Technik ist das Tragstück flexibler als die angrenzenden Schließbandstücke ausgeführt, damit bei einem Anspannen der Zuggurte die Zugkraft primär im Bereich der am Tragstück befindlichen Pelotten wirkt. Somit können aber Tragstück und angrenzenden Schließbandstücke nicht aus dem gleichen Material und einstückig hergestellt werden. Es zeigte sich nun, dass bei einer erfindungsgemäßen Rücken- oder Beckenbandage das Tragstück genauso elastisch sein kann wie die angrenzenden Bereiche der Schließbandstücke und dennoch die Kraft der Zuggurte wie gewünscht primär im Bereich des Traggurts und der Pelotten wirkt. Somit ist in vorteilhafter Weise eine Ausführungsform möglich, bei der Tragstück und angrenzenden Bereiche der Schließbandstücke aus dem gleichen Material und sogar einstückig hergestellt werden können.

Dabei kann das Tragstück bevorzugt von den angrenzenden Schließbandstücken durch jeweils eine Querversteifung abgegrenzt werden.

Es zeigte sich auch in vorteilhafter Weise, dass das Tragstück aus einem sehr leichten und luftdurchlässigen Gewirk hergestellt werden kann. Bevorzugt ist daher das Tragstück aus einem Gewirk hergestellt. Bevorzugt ist das Tragstück aus einem sehr leichten und luftdurchlässigen Gewirk hergestellt.

Bevorzugt sind das Tragstück und die am Tragstück angrenzenden Hälften der Schließbandstücke aus einem sehr leichten und luftdurchlässigen Gewirk hergestellt.

Bevorzugt weist das Tragstück einen Bereich aus einem Gewirk auf, das eine offene Struktur aufweist, die gleichzeitig sowohl in Längsrichtung als auch in Querrichtung dehnbar ist.

Bevorzugt weist das Tragstück einen Bereich aus einem Gewirk auf, das eine offene Struktur aus einer Vielzahl von Teilelementen aufweist, wobei die offene Struktur gleichzeitig sowohl in Längsrichtung als auch in Querrichtung dehnbar ist, wobei die Teilelemente der Struktur jeweils eine Breite von mindestens 2,0 mm und höchstens 10,0 mm im gedehnten Zustand haben.

Bevorzugt haben die Teilelemente der Gewirkstruktur jeweils eine Breite von mindestens 2,0 mm und höchstens 10,0 mm in gedehntem Zustand. Bevorzugt haben die Teilelemente der Gewirkstruktur jeweils eine Höhe von mindestens 2,0 mm und höchstens 10,0 mm in gedehntem Zustand.

Bevorzugt weist das Tragstück einen Bereich aus einem Gewirk auf, das gleichzeitig sowohl in Längsrichtung als auch in Querrichtung dehnbar ist, wobei das Gewirk Löcher aufweist, die jeweils eine Breite und/oder eine Höhe von mindestens 2,0 mm und höchstens 10,0 mm im gedehnten Zustand haben. Bevorzugt hat das Gewirk eine Netzstruktur.

Es zeigte sich überraschend, dass ein solches Gewirk als Hauptkomponente des Tragstücks in vorteilhafter Weise verwendet werden kann und zu einem besonders leichten und luftdurchlässigen Tragstück führt.

In vorteilhafter Weise kann ein solches Gewirk auch bei den Schließbandstücken, insbesondere bei den an das Tragstück angrenzenden Teilbereichen der Schließbandstücke verwendet werden.

Gewirke, auch Wirkwaren genannt, sind aus Fadensystemen durch Maschenbildung auf einer Wirkmaschine industriell hergestellte Stoffe. Sie gehören zu den Maschenwaren. Man unterscheidet zwischen Kulierwirkware und Kettenwirkware. Während beim Stricken oder Häkeln eine Masche neben der anderen hergestellt wird, der Faden also horizontal entlang einer Maschenreihe läuft, werden in einer Wirkware durch den Faden übereinander stehende Maschen gebildet und der Faden verläuft senkrecht und bildet mit dem benachbarten Faden ein Maschensteg. Eine Kettenwirkware wird mit vielen Fäden und mindestens ebenso vielen Nadeln hergestellt. Beim Kettenwirken laufen die Fäden vertikal und werden von den Nadeln ergriffen und durch die vorhergehende Maschenreihe gezogen.

Kettenwirkware wird beispielsweise in Form von Netzen eingesetzt, beispielsweise in Form von Gepäcknetzen in Automobilen. Die Netze können elastisch oder unelastisch ausgestaltet sein.

Bevorzugt weist das Gewirk eine offene Struktur auf, die gleichzeitig sowohl in Längsrichtung als auch in Querrichtung dehnbar ist. Bevorzugt haben die Teilelemente der offenen Struktur jeweils eine Breite von mindestens 2,0 mm bis höchstens 10,0 mm im gedehnten Zustand. Bevorzugt haben die Teilelemente der offenen Struktur alternativ oder zusätzlich jeweils eine Höhe von mindestens 2,0 mm bis höchstens 10,0 mm im gedehnten Zustand.

Erfindungsgemäß ist das Gewirk bevorzugt als Kettenwirkware ausgebildet. Erfindungsgemäß ist das Gewirk bevorzugt als Netz ausgebildet. Das Gewirk ist also bevorzugt netzförmig, d.h. sie ist ein offenporiges Flächengebilde mit regelmäßigen Maschen, die als Öffnungen ausgebildet sind. Die Öffnungen können beispielsweise rhombisch, quadratisch, sechseckig, rautenförmig oder wabenförmig ausgebildet sein. Eine Netzstruktur besteht bevorzugt aus einer Vielzahl von gleichen oder zumindest ähnlichen Teilstrukturen. Im Zusammenhang mit der vorliegenden Erfindung werden unter einer Teilstruktur eine Masche oder eine Öffnung, insbesondere ein Loch, und die die Masche oder Öffnung umgebenden und begrenzenden Fäden verstanden.

Im Zusammenhang mit der vorliegenden Erfindung wird unter einer offenen Struktur des Gewirks insbesondere eine Netzstruktur verstanden, die eine Vielzahl an Löchern und Maschen aufweist, insbesondere an größeren Löcher, bevorzugt Löcher mit einer Breite und/oder Höhe von mindestens 2,0 mm, mehr bevorzugt mindestens 3,0 mm im gedehnten Zustand. Eine offene Struktur ist also insbesondere ein offenporiges Netz.

Ausführungsmöglichkeiten eines solchen bevorzugten Gewirks kann der Fachmann der DE 10 2012 017 722 A1 entnehmen.

In dem vorliegenden Anmeldetext beziehen sich alle Angaben zur Höhe und/oder Breite der Löcher, Teilelemente und/oder Maschen auf die Höhe und/oder Breite der Löcher, Teilelemente und/oder Maschen im gedehnten Zustand des Gewirks in der Bandage, sofern nichts anderes angegeben ist.

Im Zusammenhang mit der vorliegenden Erfindung wird unter dem Begriff "im gedehnten Zustand" derjenige Zustand des Gewirks verstanden, der beim Tragen der Bandage vorliegt. Das Gewirk wird in der Bandage erfindungsgemäß bevorzugt nicht im ungedehnten Zustand eingesetzt, sondern bevorzugt in einem leicht vorgedehnten Zustand. Das Gewirk ist in der Bandage bevorzugt nicht maximal gedehnt, sondern kann bevorzugt beim Anlegen der Bandage an den Körper eines Menschen weiter gedehnt werden.

In einer bevorzugten Ausführungsform der vorliegenden Erfindung ist das Gewirk eine Kettenwirkware. In einer bevorzugten Ausführungsform weist das Gewirk keine Polfäden auf.

In einer bevorzugten Ausführungsform ist das Gewirk, das insbesondere eine Kettenwirkware ist, dadurch gekennzeichnet, dass das Gewirk keine Polfäden und eine offene Struktur aufweist, die gleichzeitig sowohl in Längsrichtung als auch in Querrichtung dehnbar ist.

In einer bevorzugten Ausführungsform weist das Gewirk einen Grundfaden und mindestens einen weiteren Faden, insbesondere Schussfaden, auf. In einer bevorzugten Ausführungsform ist die Stärke des Grundfadens größer als die Stärke des mindestens einen weiteren Fadens, insbesondere des Schussfadens.

Bei Rücken- oder Beckenbandagen aus dem Stand der Technik sind das Tragstück und die angrenzenden Schließbandstücke als Gestrick ausgeführt. Das Gewirk kann im Vergleich dazu nicht nur so ausgestaltet werden, dass es in Längs- und Querrichtung dehnbar, insbesondere gleichzeitig dehnbar ist, sondern auch, dass das Gewirk so ausgestaltet sein kann, dass es benötigten Kraft-Dehnungsvorgaben in hoher Genauigkeit entspricht, dass es also beim Anlegen einer spezifischen Kraft eine gewünschte und spezifische Dehnung aufweisen kann. Darüber hinaus ist das Gewirk durch die Löcher, bevorzugt mit einer Breite und/oder Höhe von mindestens 2 mm und höchstens 10 mm im gedehnten Zustand, atmungsaktiv. Es zeigte sich, dass das Gewirk eine deutlich bessere Atmungsaktivität aufweist als bisher im Stand der Technik verwendete Gestricke. Dadurch wird ein Wärmestau verhindert, so dass die Schweißbildung verringert wird. Das Gewirk weist eine Elastizität auf, die zu einer guten Anpassung an die Körperform führt, wobei sich die zweidimensionale Fläche dem dreidimensionalen Körper des Patienten optimal anpasst. Dies kann insbesondere durch die Ausgestaltung der Öffnungen als Rauten erreicht werden.

Auch können durch das Gewirk in vorteilhafter Weise neue Designeffekte, insbesondere Struktureffekte erzeugt werden, da Längsstreifen in unterschiedlichen Garnfarben möglich sind.

Das Gewirk ist in vorteilhafter Weise sowohl in Querrichtung als auch in Längsrichtung dehnbar.

In einer bevorzugten Ausführungsform ist die offene Struktur des Gewirks eine Rautenstruktur oder Wabenstruktur.

In einer bevorzugten Ausführungsform haben die Teilelemente der Struktur, insbesondere der Rautenstruktur oder Wabenstruktur in gedehntem Zustand, bevorzugt in maximal gedehntem Zustand jeweils eine Breite und/oder Höhe von mindestens 2 mm, bevorzugt mindestens 2 und höchstens 10 mm, bevorzugt mindestens 3 und höchstens 8 mm, bevorzugt mindestens 3 und höchstens 7 mm, bevorzugt mindestens 3 und höchstens 6 mm, bevorzugt mindestens 5 und höchstens 6 mm.

In einer bevorzugten Ausführungsform weisen die Teilelemente in Längs- und in Querrichtung die gleiche Ausdehnung auf. Bevorzugt sind im gedehnten Zustand die Höhe und die Breite der Teilelemente der in der Maschenware vorliegenden Struktur ungefähr gleich groß, bevorzugt gleich groß.

In einer bevorzugten Ausführungsform besteht das Gewirk aus einem Faden, bevorzugt einem Kettfaden mit einer Feinheit oder Stärke von mindestens 100 dtex, mehr bevorzugt von mindestens 250 dtex. In einer bevorzugten Ausführungsform besteht das Gewirk aus einem Faden, bevorzugt einem Kettfaden mit einer Feinheit oder Stärke von mindestens 500 dtex. In einer bevorzugten Ausführungsform besteht das Gewirk aus einem Faden, bevorzugt einem Kettfaden mit einer Feinheit oder Stärke von mindestens 700 dtex. In einer bevorzugten Ausführungsform besteht das Gewirk aus einem Faden, bevorzugt einem Kettfaden mit einer Feinheit oder Stärke von höchstens 10,5 ktex. In einer bevorzugten Ausführungsform besteht das Gewirk aus einem Faden, bevorzugt einem Kettfaden mit einer Feinheit oder Stärke von mindestens 900 dtex bis höchstens 999 dtex.

In einer bevorzugten Ausführungsform hat das Gewirk etwa 30% Dehnung bei einer angelegten Kraft von 1,0 bis 5,0 N/cm, bevorzugt etwa 2,5 N/cm. In einer besonders bevorzugten Ausführungsform hat das Gewirk 30% Dehnung bei einer angelegten Kraft von 1,0 bis 5,0 N/cm, bevorzugt 2,5 N/cm.

Bevorzugte Ausführungsformen ergeben sich auch aus den Unteransprüchen.

Die vorliegende Erfindung wird an folgenden Figuren und Beispielen näher erläutert, ohne dass die gezeigten Ausführungsbeispiele einschränkend zu verstehen wären.

Es zeigen
Figur 1 eine erfindungsgemäße Rücken- oder Beckenbandage mit Blickrichtung auf die Außenseite des Rückenbereichs und des Tragstücks;
Figur 2 die Bandage aus Figur 1 mit Blickrichtung auf die Innenseite des Rückenbereichs und des Tragstücks;
Figur 3 die Bandage aus Figur 2 mit angezogenen Zuggurten und miteinander angenäherten Pelotten;
Figur 4 einen Ausschnitt des Rückenbereichs mit dem Tragstück der Bandage mit Blickrichtung auf die Außenseite der Bandage (Figur 4a) und die Innenseite der Bandage (Figur 4b);
Figur 5 einen Ausschnitt einer bevorzugt für das Tragstück und die angrenzenden Teilbereiche der Schließbandstücke verwendeten Kettenwirkware mit mehreren Teilelementen. In Figur 5a ist die Kettenwirkware in einem ungedehnten Zustand gezeigt. In Figur 5b ist die Kettenwirkware in einem gedehnten Zustand gezeigt.

### Beispiele:

### Beispiel 1: Eine bevorzugte Ausführungsform der erfindungsgemäßen Rücken- oder Beckenbandage:

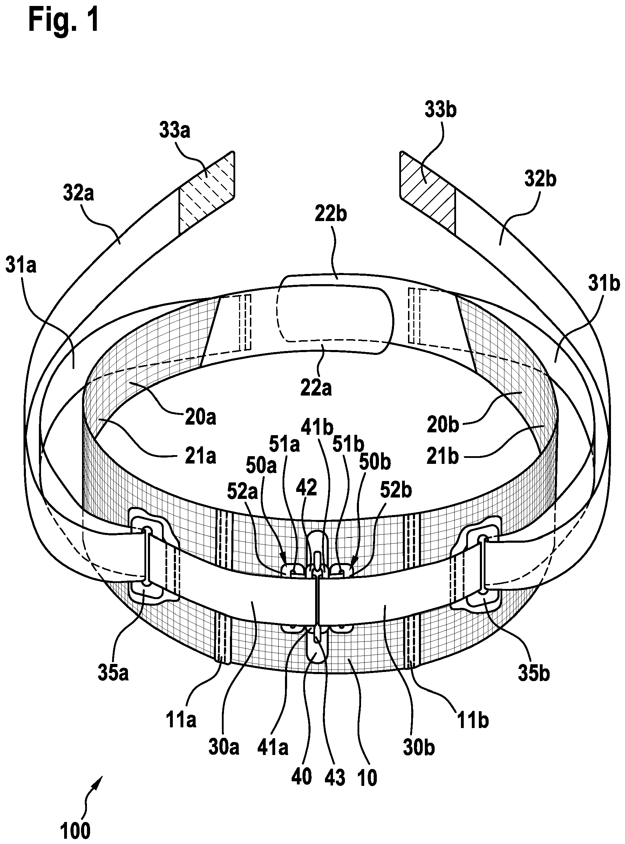

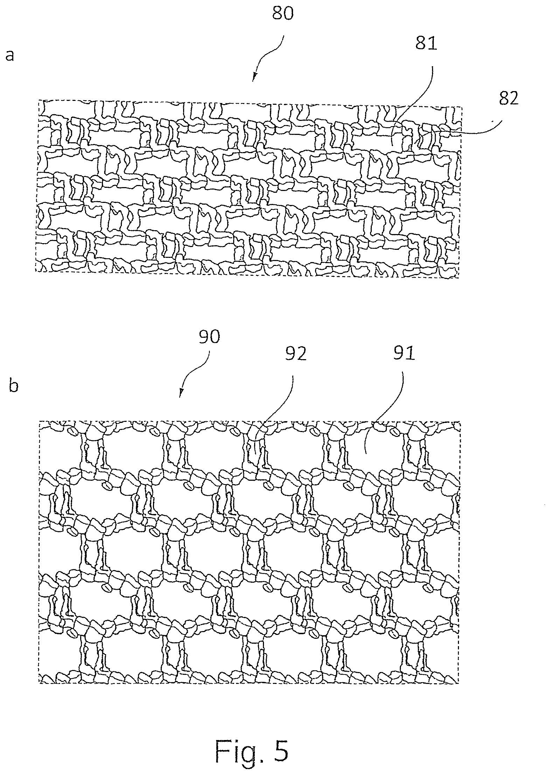

Figur 1 zeigt eine erfindungsgemäße Bandage (100) mit Blick auf die Außenseite des elastischen Tragstücks (10). An den Seiten des Tragstücks (10) schließen die beiden Schließbandstücke (20a, 20b) an. Sowohl das Tragstück (10) als auch die an dem Tragstück angrenzenden Bereiche (21a, 21b) der Schließbandstücke (20a, 20b) können in vorteilhafter Weise aus dem gleichen Material und sogar einstückig gefertigt sein, beispielsweise aus einem dehnbaren Gewirk wie es in Figur 5 gezeigt ist. Auch wenn das Tragstück (10) und die angrenzenden Bereiche (21a, 21b) der Schließbandstücke (20a, 20b) einstückig gefertigt sind, können diese dennoch durch aufgebrachte Verstärkungen in Form von Querversteifungen (11a, 11b) voneinander abgegrenzt sein. Die Schließbänder (20a, 20b) können in ihrem Endbereich (22a, 22b) miteinander verschlossen werden, beispielsweise über eine entsprechende Klettverbindung. Die Bandage (100) kann nach Anlegen derselben und Verschließen der Enden (22a, 22b) der Schließbandstücke (20a, 20b) über die Zuggurte (30a, 30b) weiter gestrafft werden. Dabei sind die Enden der Zuggurte auf der in Figur 1 dem Betrachter abgewandten Innenseite des Tragstücks (10) mit dem Tragstück verbunden. Die Zuggurte (30a, 30b) verlaufen dann auf der Innenseite zum mittleren Bereich des Tragstücks (10) und werden dort durch Öffnungen (50a, 50b) auf die Außenseite des Tragstücks (10) geführt. Die Öffnungen (50a, 50b) sind als Schlitze (51a, 51b) ausgeführt, die mit einer Verstärkung (52a, 52b) umfasst sind, sodass ein Ausreißen des Tragstückmaterials durch die Zugkräfte der Zuggurte (30a, 30b) verhindert wird. Auf der Außenseite des Tragstücks (10) werden dann die Zuggurte (30a, 30b) über Umlenkstücke (41a, 41b) umgelenkt, sodass sie in Richtung der jeweiligen Schließbandstücke (20a, 20b) verlaufen. Die Umlenkstücke (41a, 41b) werden in vorteilhafter Weise aus einem Umlenkelement (40) gebildet, das eine Umlenköse (42) umfasst, die über ein Ösenbefestigungselement (43) mit dem Tragstück (10) befestigt ist. Im Bereich der Schließbandstücke (20a, 20b) enden die Zuggurte (30a, 30b) an Ösenelementen in Form von Zugösen (35a, 35b), durch die jeweils ein Spannband (31a, 31b, 32a, 32b) hindurch gezogen ist. Die Ösenelemente (35a, 35b) sind bevorzugt nicht direkt an den Schließbandstücken (20a, 20b) befestigt. Mit den Spannbändern (32a, 32b) wird bei deren Anziehen ein Zug auf die Zuggurte (30a, 30b) nach Art eines Flaschenzugs ausgeübt, sodass die Zuggurte (30a, 30b) auf die Innenseite des Tragstücks (10) eine Zugkraft ausüben und dieses sich zusammen zieht beziehungsweise bei Lockerung der Zuggurte (31a, 31b, 32a, 32b) entspannt. Die Spannbänder (32a, 32b) können an ihren Enden (33a, 33b) miteinander verbunden werden, beispielsweise über einen Klettverschluss. Natürlich kann alternativ auch eine Ausführungsform vorgesehen sein, bei der die Zuggurte (30a, 30b) ganz nach vorne geführt werden und dort direkt angezogen und miteinander verbunden werden können.

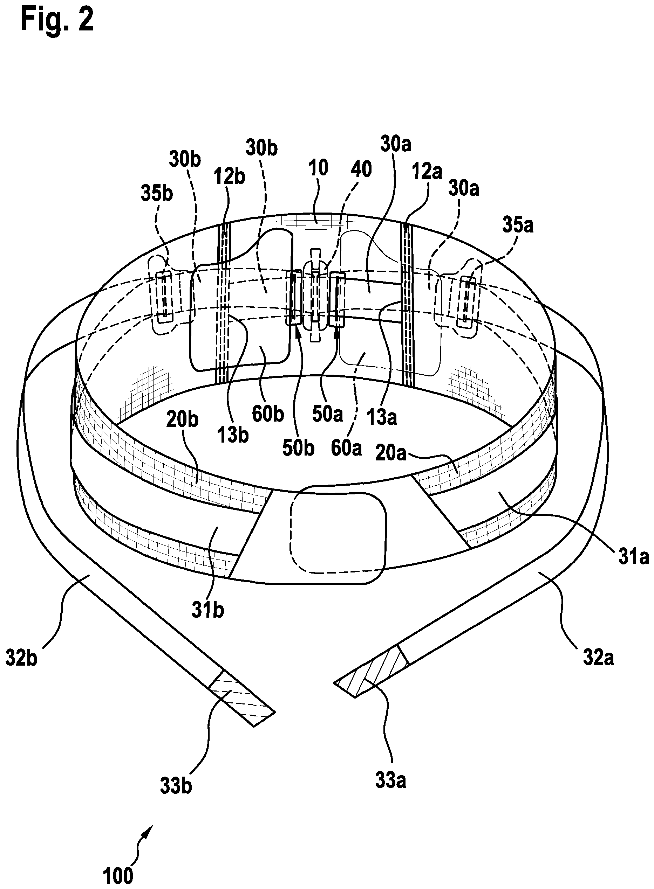

Figur 2 zeigt die Bandage (100) gemäß Figur 1 in einer Lage mit Blickrichtung auf die Innenseite des elastischen Tragstücks (10) mit den daran befestigten Pelotten (60a, 60b). Dabei ist die in Blickrichtung rechte Pelotte (60a) nur angedeutet, um den dahinter liegenden Zuggurt (30a) besser zu sehen. Das Tragstück (10) wird von den anliegenden Schließbandstücken (20a, 20b) durch eine Verstärkung (12a, 12b) abgegrenzt, die aber Bestandteil des Tragstücks (10) ist. Dabei dienen diese Verstärkungen in vorteilhafter Weise als Klettverbindungselemente für die Pelotten (60a, 60b), so dass dort die Pelotten (60a, 60b) mittels einer Klettverbindung an dem Tragstück (10) befestigt werden können. In diesen Pelotten-Befestigungsbereichen (12a, 12b) sind jeweils auch die Zuggurte (30a, 30b) an Ansatzbereichen (13a, 13b) mit dem Tragstück (10) befestigt. Die Zuggurte (30a, 30b) werden dann auf der Innenseite des Tragstücks (10) zu den Öffnungen (50a, 50b) und von dort auf die Außenseite des Tragstücks (10) geführt. Dort werden sie durch das Umlenkelement (40) umgelenkt und zu den Ösenelementen in Form von Zugösen (35a, 35b) geführt, wo sie wie für Figur 1 bereits beschrieben mit den Spannbändern (31a, 31b, 32a, 32b) verbunden sind, wobei die Spannbänder an ihren Enden (33a, 33b) über eine Klettverbindung miteinander verschließbar sind.

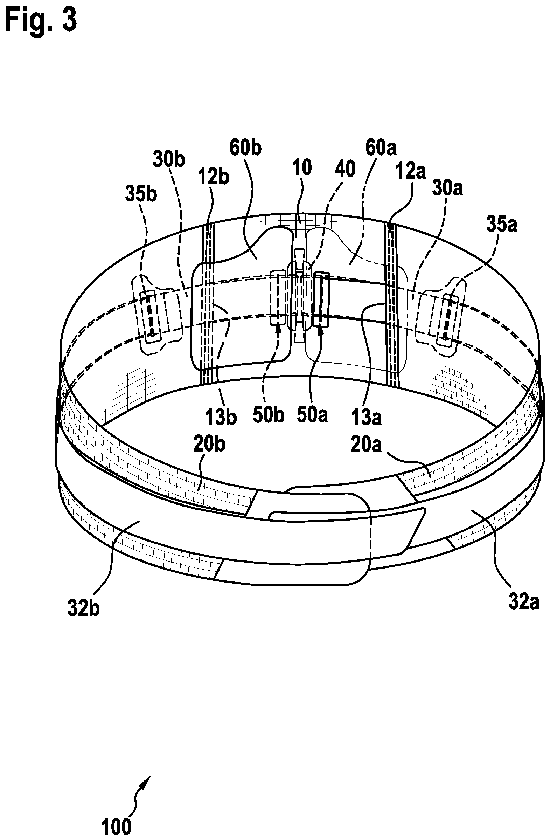

Figur 3 zeigt die Bandage (100) aus Figur 2 mit dem Tragstück (10) und den Schließbandstücken (20a, 20b) in geschlossenem Zustand. Dabei sind auch die Spannbänder (32a, 32b) angezogen und miteinander verbunden. Sie üben somit über die Ösenelemente (35a, 35b) eine Zugkraft auf die Zuggurte (30a, 30b) aus. Durch die Umlenkung der Zuggurte (30a, 30b) über das Umlenkelement (40) und die nachfolgende Führung der Zuggurte (30a, 30b) durch die Öffnungen (50a, 50b) auf die Innenseite des Tragstücks (10) wirkt diese Zugkraft direkt auf die Ansatzbereiche (13a, 13b) und die dort beispielsweise über Klettverbindungen an Befestigungsbereichen (12a, 12b) angebrachten Pelotten (60a (hier nur angedeutet), 60b), sodass die Pelotten stärker zur Mitte des Tragstücks (10) zusammengezogen werden. Bei dieser Zugspannung der beiden Zuggurte (30a, 30b) ergibt sich also auf Grund der Richtungsumkehr über das Umlenkelement (40) eine Zusammenziehung des Tragstücks (10) und damit eine entsprechende Annäherung der daran angebrachten Pelotten (60a, 60b). Dabei wirkt die Zugkraft der Zuggurte (30a, 30b) in vorteilhafter Weise direkt auf der Innenseite des Tragstücks (10) und dabei auf den Bereich (12a, 12b) des Tragstücks (10), an dem die Pelotten (60a, 60b) befestigt sind.

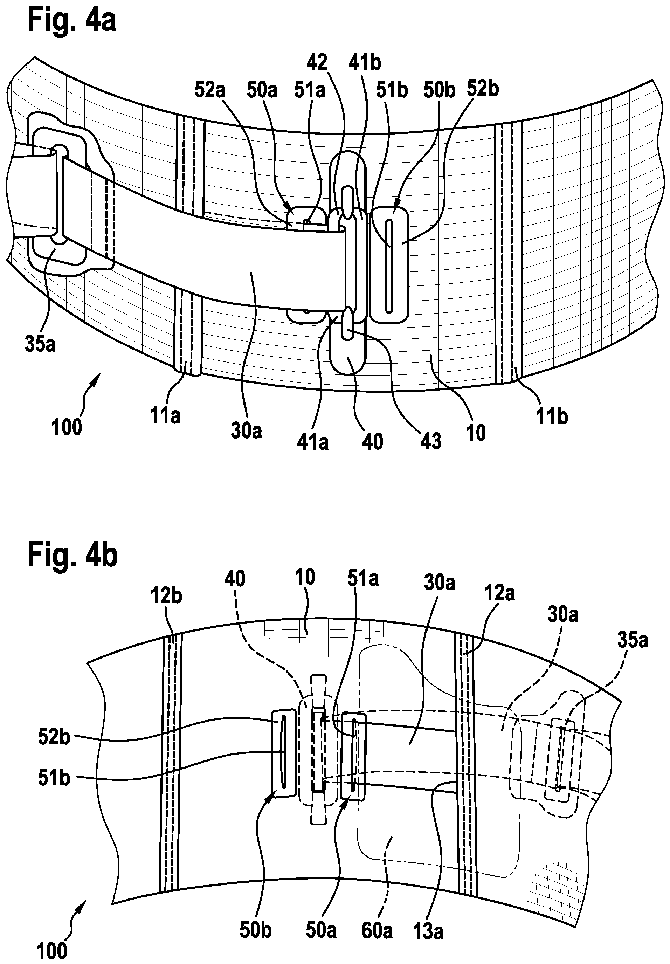

Figur 4 zeigt einen vergrößerten Ausschnitt der Bandage (100) im Bereich des Tragstücks (10). Dabei ist die Bandage (100) in Figur 4a von außen und in Figur 4b von innen zu sehen. Zur besseren Darstellung wurde dabei nur ein Zuggurt (30a) mit zugehörigem Ösenelement (35a) gezeigt. Aus den Figuren 1 bis 3 ergibt sich der Verlauf des hier nicht gezeigten zweiten Zuggurts.

In Figur 4a sind wieder die Verstärkungselemente (11a, 11b), die das Tragstück (10) von den anliegenden Schließbandstücken abgrenzen, zu sehen. Das Umlenkelement (40) umfasst wieder eine Umlenköse (42), die die zwei Umlenkstücke (41a, 41b) bildet und über ein Ösenbefestigungselement (43) mit dem Tragstück (10) befestigt ist. Die Öffnungen (50a, 50b), durch die die Zuggurte (gezeigt 30a) auf die Innenseite des Tragstücks (10) geführt werden, sind aus Schlitzen (51a, 51b) gebildet, die mit einer Verstärkung (52a, 52b) umrandet sind.

In Figur 4b ist das Umlenkelement (40) nur angedeutet, da es auf der Außenseite des Tragstücks (10) liegt. Durch die mit einer Umrandung (52b) versehenen Schlitze (51a, 51b) ausgebildeten Öffnungen (50a, 50b) werden die Zuggurte (gezeigt hier 30a) nach innen geführt, wo sie über Ansatzbereiche (13a) mit dem Tragstück (10) verbunden sind. In diesen Ansatzbereichen befinden sich auch die Befestigungsbereiche (12a, 12b) der Pelotten (hier angedeutet 60a), die beispielsweise über Klettverbindungen an dem Tragstück (10) befestigt werden können.

### Beispiel 2: Kettenwirkware für das Tragstück und die angrenzenden Teilbereiche der Schließbandstücke

Es wurde eine Kettenwirkware aus einem doppelt umwundenen Rohgummifaden maschinell hergestellt. Der verwendete maschenbildende Faden hatte einen Titer von 940 dtex. Von dieser Kettenwirkware (80, 90) ist in der Figur 5 ein Ausschnitt zu sehen. Die Kettenwirkware (80, 90) ist netzförmig. Die Kettenwirkware (80, 90) besteht aus einer Vielzahl von Teilelementen, wobei jedes Teilelement aus einer Öffnung (81, 91) und dem die Öffnung (81, 91) umgebenden Faden (82, 92) besteht, der die Öffnung (81, 91) auch gleichzeitig begrenzt und somit die Größe der Öffnung vorgibt. In Figur 5a ist die Kettenwirkware (80) nicht gedehnt. In Figur 5b ist die Kettenwirkware (90) mit einer bestimmten Kraft gedehnt. Durch die Dehnung der Kettenwirkware (90) öffnet sich die Struktur (92), so dass sich die Öffnungen (91) in Dehnungsrichtung vergrößern. Eine solche Kettenwirkware verbessert den Tragekomfort der erfindungsgemäßen Rücken- oder Beckenbandage durch das reduzierte Gewicht und die luftdurchlässigen Öffnungen (91).

## Patentansprüche

1. Rücken- oder Beckenbandage (100) mit benachbarten Pelotten (60a, 60b), die auf der Innenseite eines Tragstücks (10) im Rückenbereich der Bandage (100) angebracht sind, wobei an den gegenüberliegenden Randbereichen des Tragstücks (10) jeweils ein Schließbandstück (20a, 20b) zum Anlegen der Bandage (100) durch Zusammenfassen der Enden (22a, 22b) der beiden Schließbandstücke (20a, 20b) ansetzt, wobei dem Tragstück (10) zwei Zuggurte (30a, 30b) zugeordnet sind, die derart geführt sind, dass die Pelotten (60a, 60b) bei Spannung der Zuggurte (30a, 30b) sich annähern und auf das betreffende Körperteil einen von einer auf die Zuggurte (30a, 30b) wirkenden Zugkraft abhängigen Druck ausüben, wobei die Zuggurte (30a, 30b) im Bereich der Pelotten (60a, 60b) mit dem Tragstück (10) verbunden sind, **dadurch gekennzeichnet, dass** die Zuggurte (30a, 30b) auf der Innenseite des Tragstücks (10) mit dem Tragstück (10) verbunden sind.

2. Rücken- oder Beckenbandage nach Anspruch 1, wobei die Zuggurte (30a, 30b) teilweise auf der Außenseite des Tragstücks (10) verlaufen und das Tragstück (10) mindestens eine Öffnung (50a, 50b) aufweist, durch die die Zuggurte (30a, 30b) auf die Innenseite des Tragstücks (10) geführt werden.

3. Rücken- oder Beckenbandage nach einem der vorhergehenden Ansprüche, wobei der erste Zuggurt (30a) durch eine erste Öffnung (50a) im Tragstück (10) auf die Innenseite des Tragstücks (10) geführt wird und der zweite Zuggurt (30b) durch eine zweite Öffnung (50b) im Tragstück (10) auf die Innenseite des Tragstücks (10) geführt wird.

4. Rücken- oder Beckenbandage nach einem der vorhergehenden Ansprüche, wobei die mindestens eine Öffnung (50a, 50b) ein Schlitz (51a, 51b) im Tragstück (10) ist.

5. Rücken- oder Beckenbandage nach einem der vorhergehenden Ansprüche, wobei die Zuggurte (30a, 30b) je über ein an dem Tragstück (10) angebrachten Umlenkstück (41a, 41b) unter Richtungsumkehr von dem Bereich der Pelotten (60a, 60b) zu dem jeweiligen Randbereich des Tragstücks (10) geführt sind.

6. Rücken- oder Beckenbandage nach Anspruch 5, wobei die Umlenkstücke (41a, 41b) auf der Außenseite des Tragstücks (10) befestigt ist.

7. Rücken- oder Beckenbandage nach einem der Ansprüche 5 oder 6, wobei eine in der Mitte des Tragstücks befestigte Umlenköse (40) die zwei Umlenkstücke (41a, 41b) bildet.

8. Rücken- oder Beckenbandage nach einem der vorhergehenden Ansprüche, wobei jeweils ein Zuggurt (30a, 30b) an jeweils einem Befestigungsbereich (13a, 13b) einer Pelotte (60a, 60b) mit dem Tragstück (10) verbunden ist.

9. Rücken- oder Beckenbandage nach einem der vorhergehenden Ansprüche, wobei das Tragstück (10) einen Bereich aus einem Gewirk (80, 90) aufweist, das eine offene Struktur aus einer Vielzahl von Teilelementen (81, 82, 91, 92) aufweist, wobei die offene Struktur gleichzeitig sowohl in Längsrichtung als auch in Querrichtung dehnbar ist, wobei die Teilelemente (81, 82, 91, 92) der Struktur jeweils eine Breite von mindestens 2,0 mm und höchstens 10,0 mm im gedehnten Zustand haben.

10. Rücken- oder Beckenbandage nach einem der vorhergehenden Ansprüche, wobei das Tragstück (10) und die am Tragstück (10) angrenzende Hälfte (21a, 21b) der Schließbandstücke (20a, 20b) die gleiche Elastizität aufweisen.

## Claims

1. A bandage for the back or pelvis (100) having adjacent pads (60a, 60b), which are mounted on the inner face of a supporting piece (10) in the back area of the bandage (100), wherein respectively one fastener strap piece (20a, 20b) attaches at the opposite edge regions of the supporting piece (10) for applying the bandage (100) by joining together the ends (22a, 22b) of the two fastener strap pieces (20a, 20b), wherein two tensioning belts (30a, 30b) are assigned to the supporting piece (10), which are guided in such a manner that the pads (60a, 60b) approach each other when tensioning the tensioning belts (30a, 30b) and apply a pressure dependent on a tension force acting on the tensioning belts (30a, 30b) onto the relevant body part, wherein the tensioning belts (30a, 30b) are connected to the supporting piece (10) in the area of the pads (60a, 60b), **characterised in that** the tensioning belts (30a, 30b) are connected to the supporting piece (10) on the inner face of the supporting piece (10).

2. The bandage for the back or pelvis as recited in claim 1, wherein the tensioning belts (30a, 30b) run partially on the outer face of the supporting piece (10) and the supporting piece (10) has at least one opening (50a, 50b) through which the tensioning belts (30a, 30b) are guided to the inner face of the supporting piece (10).

3. The bandage for the back or pelvis as recited in any one of the preceding claims, wherein the first tensioning belt (30a) is guided through a first opening (50a) in the supporting piece (10) to the inner face of the supporting piece (10) and the second tensioning belt (30b) is guided through a second opening (50b) in the supporting piece (10) to the inner face of the supporting piece (10).

4. The bandage for the back or pelvis as recited in any one of the preceding claims, wherein the at least one opening (50a, 50b) is a slit (51a, 51b) in the supporting piece (10).

5. The bandage for the back or pelvis as recited in any one of the preceding claims, wherein the tensioning belts (30a, 30b) are each guided via a deflection piece (41a, 41b) mounted at the supporting piece (10) by reversing the direction from the area of the pads (60a, 60b) to the respective edge region of the supporting piece (10).

6. The bandage for the back or pelvis as recited in claim 5, wherein the deflection pieces (41a, 41b) are attached on the outer face of the supporting piece (10).

7. The bandage for the back or pelvis as recited in one of claims 5 or 6, wherein a deflection lug (40) attached in the centre of the supporting piece forms the two deflection pieces (41a, 41b).

8. The bandage for the back or pelvis as recited in any one of the preceding claims, wherein respectively one tensioning belt (30a, 30b) is connected in respectively one attachment area (13a, 13b) of a pad (60a, 60b) to the supporting piece (10).

9. The bandage for the back or pelvis as recited in any one of the preceding claims, wherein the supporting piece (10) has an area made from a knitted fabric (80, 90) which has an open structure made from a plurality of partial elements (81, 82, 91, 92), wherein the open structure simultaneously is stretchable in the longitudinal direction as well as also in the lateral direction, wherein the partial elements (81, 82, 91, 92) of the structure respectively have a width of at least 2.0 mm and no more than 10.0 mm in the stretched state.

10. The bandage for the back or pelvis as recited in any one of the preceding claims, wherein the supporting piece (10) and the half (21a, 21b) of the fastener strap pieces (20a, 20b) adjacent to the supporting piece (10) have equal elasticity.

## Revendications

1. Bandage dorsal ou pelvien (100) avec des pelotes adjacentes (60a, 60b) montées au niveau du côté intérieur d'une pièce de portage (10) dans la région dorsale du bandage (100), dans lequel respectivement une pièce de bande de fermeture (20a, 20b) se rattache au niveau des régions de bord opposées de la pièce de portage (10), pour appliquer le bandage (100) enjoignant les extrémités (22a, 22b) des deux pièces de bande de fermeture (20a, 20b), dans lequel deux sangles de tension (30a, 30b) sont associées à la pièce de portage (10), les sangles étant guidées de telle manière que les pelotes (60a, 60b) s'approchent l'une à l'autre lorsque les sangles de tension (30a, 30b) sont tendues, et exercent une pression sur la partie de corps respective en fonction d'une force de traction agissant sur les sangles de tension (30a, 30b), les sangles de tension (30a, 30b) étant reliées à la pièce de portage (10) dans la région des pelotes (60a, 60b), **caractérisé en ce que** les sangles de tension (30a, 30b) sont reliées avec la pièce de portage (10) au niveau du côté intérieur de la pièce de portage (10).

2. Bandage dorsal ou pelvien selon la revendication 1, dans lequel les sangles de tension (30a, 30b) s'étendent partiellement sur le côté extérieur de la pièce de portage (10), et la pièce de portage (10) comporte au moins une ouverture (50a, 50b) par laquelle les sangles de tension (30a, 30b) sont guidées vers le côté intérieur de la pièce de portage (10).

3. Bandage dorsal ou pelvien selon l'une quelconque des revendications précédentes, dans lequel la première sangle de tension (30a) est guidée vers le côté intérieur de la pièce de portage (10) par une première ouverture (50a) dans la pièce de portage (10), et la seconde sangle de tension (30b) est guidée vers le côté intérieur de la pièce de portage (10) par une seconde ouverture (50b) dans la pièce de portage (10).

4. Bandage dorsal ou pelvien selon l'une quelconque des revendications précédentes, dans lequel l'au moins une ouverture (50a, 50b) est une fente (51a, 51b) dans la pièce de portage (10).

5. Bandage dorsal ou pelvien selon l'une quelconque des revendications précédentes, dans lequel les sangles de tension (30a, 30b) sont respectivement guidées à travers une pièce de renvoi (41a, 41b) montée sur la pièce de portage (10), en changeant l'orientation à partir de la région des pelotes (60a, 60b) jusqu'à la région de bord respective de la pièce de portage (10).

6. Bandage dorsal ou pelvien selon la revendication 5, dans lequel les pièces de renvoi (41a, 41b) sont fixées sur le côté extérieur de la pièce de portage (10).

7. Bandage dorsal ou pelvien selon l'une quelconque des revendications 5 ou 6, dans lequel les deux pièces de renvoi (41a, 41b) sont formées par un oeillet de renvoi (40) monté au milieu de la pièce de portage.

8. Bandage dorsal ou pelvien selon l'une quelconque des revendications précédentes, dans lequel respectivement une sangle de tension (30a, 30b) est reliée en chaque cas avec la pièce de portage (10) au niveau d'une région de fixation (13a, 13b) d'une pelote (60a, 60b).

9. Bandage dorsal ou pelvien selon l'une quelconque des revendications précédentes, dans lequel la pièce de portage (10) comporte une région en tissu tricoté (80, 90) avec une structure ouverte d'une pluralité d'éléments partiels (81, 82, 91, 92), la structure ouverte étant expansible soit dans la direction longitudinale soit dans la direction transversale, les éléments partiels (81, 82, 91, 92) de la structure respectivement ayant une largeur d'au moins 2,0 mm et d'au plus 10,0 mm dans l'état expandu.

10. Bandage dorsal ou pelvien selon l'une des revendications précédentes, dans lequel la pièce de portage (10) et la moitié (21a, 21b) des pièces de bande de fermeture (20a, 20b) adjacentes à la pièce de portage (10) ont la même élasticité.
